# EUROPÄISCHE PATENTSCHRIFT

(11) **EP 0 423 658 B1**
(45) Veröffentlichungstag und Bekanntmachung des Hinweises auf die Patenterteilung: **18.01.1995**
(21) Anmeldenummer: 90119650.1
(22) Anmeldetag: 13.10.1990
(51) Int. Cl.: C07C 69/15, C07C 67/54, C07C 67/055, C07C 67/58

(54) **Verfahren zur Isolierung von Vinylacetat**
Process for isolating vinyl acetate
Procédé d'isolement de l'acétate de vinyl

(30) Priorität: 17.10.1989 DE 3934614
(43) Veröffentlichungstag der Anmeldung: 24.04.1991
(73) Patentinhaber: HOECHST AKTIENGESELLSCHAFT, 65926 Frankfurt am Main (DE)
(72) Erfinder: Roscher, Günter, Dr., D-6233 Kelkheim (Taunus) (DE); Schmidt, Karl-Heinz, Dr., D-6270 Idstein/Taunus (DE); Eichler, Klaus, Dr., D-6236 Eschborn (DE); Hörstermann, Peter, Dr., D-6240 Königstein/Taunus (DE); Gradl, Reinhard, Dr., D-5042 Erftstadt (DE); Langner, Horst, D-6234 Hattersheim (DE)

(56) Entgegenhaltungen:
- DE-A- 3 422 575

## Beschreibung

Die Herstellung von Vinylacetat durch Umsetzung von Ethylen mit Essigsäure und Sauerstoff oder sauerstoffhaltigen Gasen an Festbettkatalysatoren in der Gasphase ist bereits bekannt. Die Reaktion erfolgt im allgemeinen bei Drucken von 1 bis 25 bar und Temperaturen von 100 bis 250°C. Geeignete Katalysatoren enthalten einen Edelmetallanteil und einen Aktivatoranteil. Der Edelmetallanteil besteht aus Palladium und/oder dessen Verbindungen; zusätzlich können noch Gold oder dessen Verbindungen anwesend sein. Der Aktivatoranteil besteht aus Verbindungen von Elementen der 1. Hauptgruppe und/oder der 2. Hauptgruppe und/oder Cadmium. Diese aktiven Komponenten werden auf Träger in feiner Verteilung aufgebracht, wobei als Trägermaterial im allgemeinen Kieselsäure oder Aluminiumoxid verwendet wird.

Im allgemeinen liegt der Palladiumgehalt im Katalysator zwischen 0,5 und 5 Gew.-%.

Wird Gold bzw. eine seiner Verbindungen eingesetzt, so wird es in einem Anteil von 0,01 bis 4 Gew.-% zugegeben.

Jeder einzelne Aktivator wird im allgemeinen ebenfalls in einem Anteil von 0,01 bis 4 Gew.-% zugegeben. Bei allen drei Prozentangaben wird jeweils der Metallanteil der Komponente auf die Gesamtmasse des Trägerkatalysators bezogen. Bevorzugt sind folgende Katalysatoren:

Palladium/Alkalielement/Cadmium sowie Palladium/Gold/Alkalielement, wobei Palladium bzw. Gold als Metalle oder Verbindungen im fertigen Katalysator vorliegen können und wobei als Alkalielement Kalium bevorzugt ist (in Form eines Carboxylats).

Besonders bevorzugt sind die Katalysatoren Palladiumacetat/Kaliumacetat/Cadmiumacetat, sowie Palladiumacetat/Bariumacetoaurat/Kaliumacetat.

In dem mehrstufigen katalytischen Prozess entstehen Vinylacetat und Wasser in äquimolaren Mengen, wie in der folgenden Bruttogleichung dargestellt: 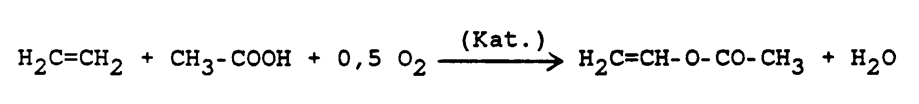
Aus der nicht ganz zu vermeidenden Totaloxidation des Ethylens entsteht CO₂ und Wasser:

H₂C=CH₂ + 3 O₂ → 2 CO₂ + 2 H₂O.

Es fällt daher mehr als 1 mol Wasser pro mol Vinylacetat an; im allgemeinen macht das Gewicht des Wassers ca. ein Viertel des Gewichts des gebildeten Vinylacetats aus.

Außer CO₂ bilden sich in geringer Menge noch andere Nebenprodukte, darunter auch Ethylacetat in einem Anteil von etwa 1000 - 2000 Gew.-ppm, bezogen auf das gebildete Vinylacetat. Ethylacetat darf in reinem Vinylacetat nur in einem Anteil von höchstens 150 Gew.-ppm enthalten sein. Die Abtrennung des Ethylacetats erforderte bisher einen hohen Energieaufwand. Gesucht wurde daher ein Verfahren, das Vinylacetat mit einem geringeren Aufwand an Energie als bisher in reiner Form und frei von Ethylacetat und anderen Nebenprodukten liefert.

Gegenstand der Erfindung ist nun ein Verfahren, mit dem Vinylacetat mit geringerem Energieverbrauch isoliert und insbesondere von Ethylacetat befreit werden kann.

Das zur Reaktion eingesetzte Gemisch enthält ein mehrfaches der stöchiometrisch erforderlichen Menge an Ethylen.

Dementsprechend ist der Ethylenumsatz mit etwa 10 % relativ gering und das nicht umgesetzte Ethylen muß in die Reaktionszone zurückgeführt werden. Das Vinylacetat wird üblicherweise in einem mehrstufigen Verfahren aus dem Gemisch der gasförmig anfallenden Reaktionsprodukte abgetrennt.

Bei dem Verfahren gemäß DE-OS 3 422 575 (= US-PS 4 818 347) wird das den Vinylacetatreaktor verlassende heiße Gasgemisch, das im wesentlichen aus nicht umgesetztem Ethylen, nicht umgesetzter Essigsäure, nicht umgesetztem Sauerstoff, Stickstoff, Argon, Vinylacetat, Reaktionswasser, CO₂, sowie Ethylacetat besteht, in eine ohne zusätzliche Beheizung arbeitende Destillationskolonne eingeleitet, die sogenannte Vorentwässerungskolonne. Das am Kopf dieser Kolonne austretende Gasgemisch wird auf -20 bis +50°C abgekühlt, wobei es zum Teil kondensiert. Das Kondensat trennt sich in zwei Phasen, eine organische und eine wäßrige. Die wäßrige Phase wird abgezogen, die organische Phase wird ganz oder teilweise als Rücklauf auf den Kopf der Vorentwässerungskolonne zurückgeführt. Der nichtkondensierte Teil des Kopfdampfes dieser Kolonne enthält noch gasförmiges Vinylacetat. Dieses wird in einer mit Essigsäure als Waschflüssigkeit betriebenen Waschkolonne aus dem Gasgemisch herausgewaschen. Das verbleibende Restgas wird zurückgeführt zum Reaktor. Am Sumpf der Vorentwässerungskolonne fällt ein Gemisch an, das aus Vinylacetat, Essigsäure und etwa der Hälfte des Reaktionswassers sowie Nebenprodukten besteht. Die andere Hälfte des Reaktionswassers ist bereits ohne Energiezufuhr abgetrennt worden und bildet die wäßrige Phase des Kondensats, das bei der oben erwähnten Abkühlung des Kopfdampfes der Vorentwässerungskolonne entsteht.

Das Sumpfprodukt der Vorentwässerungskolonn wird in einer zweiten Kolonne getrennt in A) wassergesättigtes Vinylacetat als Kopfprodukt, B) einen ethylacetathaltigen Seitenstrom, der abgezogen wird und C) ein Sumpfprodukt, das als Rückessigsäure ins System zurückgeführt wird. Das wassergesättigte Vinylacetat A) wird dann mit dem Sumpfprodukt der Waschkolonne zusammengeführt, und das Gemisch wird in zwei weiteren Kolonnen aufgearbeitet.

Es wurde nun überraschenderweise gefunden, daß es vorteilhafter ist, das Sumpfprodukt der Kreisgaswäsche nicht unmittelbar mit dem wassergesättigten Vinylacetat A) zusammenzuführen, sondern es zuerst in eine weitere Kolonne einzuleiten, die als Kopfprodukt ein Vinylacetat-Wasser-Azeotrop liefert und als Sumpfprodukt wäßrige Essigsäure (die in die oben erwähnte zweite Kolonne oder in die Kreisgaswäsche oder direkt in die Reaktionszone zurückgeführt wird). Das Kopfprodukt wird nach Abtrennung der durch Abkühlung gebildeten wäßrigen Phase zusammen mit dem wassergesättigten Vinylacetat A) (Kopfprodukt der zweiten Kolonne) in einer weiteren Kolonne getrocknet, und in einer letzten Kolonne wird reines Vinylacetat über Kopf destilliert. Diese Aufarbeitungsmethode hat einen ähnlich geringen Aufwand an Destillationsenergie wie die in der DE-OS 3 422 575 (= US-PS 4 818 347) beschriebene, jedoch den Vorteil, daß für die Aufarbeitung insgesamt eine geringere Anzahl an Kolonnenböden erforderlich ist, was erheblich weniger Investitionskosten bedeutet.

Die Erfindung betrifft somit ein Verfahren zur Isolierung von Vinylacetat aus einem Vinylacetat, Ethylacetat, Essigsäure, Wasser und Kohlendioxid enthaltenden Gasgemisch, welches bei der Reaktion von Ethylen mit Essigsäure und Sauerstoff in einer Reaktionszone in der Gasphase an Palladium oder Palladiumverbindungen enthaltenden Katalysatoren gebildet wird, wobei man
a) das aus der Reaktionszone austretende Gasgemisch in eine erste Destillationskolonne einleitet,
b) das am Kopf der ersten Destillationskolonne austretende Gasgemisch auf -20 bis +50°C abkühlt, wobei sich das anfallende Kondensat in eine wäßrige und eine organische Phase trennt,
c) die in Schritt b) gebildete wäßrige Phase abzieht,
d) die in Schritt b) gebildete organische Phase ganz oder teilweise als Rücklauf auf den Kopf der in Schritt a) benutzten ersten Destillationskolonne zurückführt und einen etwaigen nicht als Rücklauf verwendeten Teil der organischen Phase abzieht,
e) das im Schritt b) nicht kondensierte, Vinylacetat enthaltende Gas in einer Waschkolonne mit mindestens 90 %iger wäßriger Essigsäure wäscht und dabei am Sumpf eine vinylacetathaltige essigsaure Lösung gewinnt,
f) das Vinylacetat, Ethylacetat, Essigsäure und Wasser enthaltende Sumpfprodukt von Schritt a) einer zweiten Destillationskolonne zuführt und aus einer Anreicherungszone oberhalb von deren Sumpf einen ethylacetathaltigen Seitenstrom abzieht,
g) das Essigsäure und Wasser enthaltende Sumpfprodukt von Schritt f) ganz oder teilweise zur Gaswäsche in Schritt e) benutzt,
h) den Kopfdampf von Schritt f) abkühlt, wobei sich das anfallende Kondensat in eine wäßrige und eine organische Phase trennt,
i) die in Schritt h) gebildete wäßrige Phase abzieht,
k) einen Teil der in Schritt h) gebildeten organischen Phase als Rücklauf auf den Kopf der in Schritt f) benutzten zweiten Destillationskolonne zurückführt,
l) den restlichen Teil der in Schritt h) gebildeten organischen Phase abzieht,
dadurch gekennzeichnet, daß man
m) das Sumpfprodukt der in Schritt e) benutzten Waschkolonne in eine dritte Destillationskolonne einleitet,
n) das Sumpfprodukt der in Schritt m) benutzten dritten Destillationskolonne in die in Schritt f) benutzte zweite Destillationskolonne oder in die in Schritt e) benutzte Waschkolonne oder in die Reaktionszone zurückführt,
o) den Kopfdampf von Schritt m) abkühlt, wobei sich das anfallende Kondensat in eine wäßrige und eine organische Phase trennt,
p) die in Schritt o) gebildete wäßrige Phase abzieht,
q) einen Teil der in Schritt o) gebildeten organischen Phase als Rücklauf auf den Kopf der in Schritt m) benutzten dritten Destillationskolonne zurückführt,
r) den restlichen Teil der in Schritt o) gebildeten organischen Phase zusammen mit der in Schritt l) abgezogenen restlichen organischen Phase und zusammen mit der etwaigen, in Schritt d) abgezogenen restlichen organischen Phase in eine vierte Destillationskolonne einleitet,
s) den Kopfdampf von Schritt r) abkühlt, wobei sich das anfallende Kondensat in eine wäßrige und eine organiche Phase trennt,
t) die in Schritt s) gebildete wäßrige Phase abzieht,
u) die in Schritt s) gebildete organische Phase ganz oder teilweise als Rücklauf auf den Kopf der in Schritt r) benutzten vierten Destillationskolonne zurückführt und einen etwaigen nicht als Rücklauf verwendeten Teil der organischen Phase zur Leichtsiederabtrennung abzieht,
v) das Sumpfprodukt von Schritt r) in eine fünfte Destillationskolonne einleitet,
w) am Kopf der in Schritt v) verwendeten fünften Destillationskolonne reines Vinylacetat abzieht.

In Schritt a) wird vorzugsweise das aus der Reaktionszone austretende Gasgemisch zunächst im Gegenstromwärmeaustausch mit dem kälteren Kreisgas (das damit aufgeheizt und dann zur Reaktion zurückgeführt wird) auf etwa 115 - 130°C abgekühlt (wobei noch keine Kondensation der verflüssigbaren Anteile eintritt) und erst dann in die erste Destillationskolonne eingeleitet.

Die Menge der in Schritt b) gebildeten organischen Phase ist abhängig davon, bis zu welcher Temperatur in diesem Schritt abgekühlt wird. Denjenigen Teil der organischen Phase aus Schritt b), den man in Schritt d) nicht als Rücklauf benutzt, leitet man in Schritt r) (zusammen mit der nicht als Rücklauf für die zweite bzw. dritte Destillationskolonne benutzten organischen Phase aus Schritt h) und o)) in die vierte Destillationskolonne ein. Vorzugsweise wählt man die Abkühlungstemperatur in Schritt b) und den als Rücklauf in Schritt d) benutzten Anteil der in b) gebildeten organischen Phase derart, daß möglichst wenig Vinylacetat, aber möglichst das gesamte Ethylacetat im Sumpfprodukt von Schritt a) enthalten sind. Dies bedeutet, daß sich etwa 20 bis 50 Gew.-% des Vinylacetats in diesem Sumpfprodukt befinden. Die anderen 50 - 80 Gew.-% des Vinylacetats befinden sich dann teilweise in der in Schritt e) gebildeten essigsauren Lösung und teilweise in dem nicht als Rücklauf in Schritt d) benutzten Anteil der in Schritt b) gebildeten organischen Phase.

In der Gaswäsche von Schritt e) setzt man zumindest teilweise das Sumpfprodukt der zweiten Destillationskolonne (Schritt f)) ein, zusätzlich kann man das in Schritt m) gebildete Sumpfprodukt der dritten Destillationskolonne einsetzen; beide Sumpfprodukte bestehen hauptsächlich aus Essigsäure und enthalten höchstens 10 Gew.-% Wasser. Ein nicht in Schritt e) benötigter Anteil der genannten Sumpfprodukte wird vorzugsweise zum Reaktor zurückgeführt, nachdem ein kleiner Teil zur Entfernung von Hochsiedern und Polymeren ausgeschleust wurde.

In Schritt k) führt man vorzugsweise nur soviel von der in Schritt h) gebildeten organischen Phase als Rücklauf zurück, daß der Kopfdampf der zweiten Destillationskolonne möglichst wenig Essigsäure und Ethylacetat enthält. Denjenigen Teil der organischen Phase, der nicht für diesen Zweck benötigt wird, leitet man gemäß Schritt r) in die vierte Destillationskolonne ein.

In Schritt q) führt man vorzugsweise soviel von der in Schritt o) gebildeten organischen Phase als Rücklauf auf den Kopf der dritten Destillationskolonne zurück, daß in deren Sumpf möglichst wenig Vinylacetat erhalten ist.

In Schritt u) wird vorzugsweise die in Schritt s) gebildete organische Phase nicht ganz als Rücklauf für die vierte Destillationskolonne benutzt, sondern ein zur Leichtsiederabtrennung ausreichender Teil abgezogen.

Der überraschende Vorteil des erfindungsgemäßen Verfahrens besteht in der energiesparenden Abtrennung des bei der Vinylacetatreaktion als Nebenprodukt gebildeten Ethylacetats bei geringerem Apparateaufwand als bisher (DE-OS 3 422 575 bzw. US-PS 4 818 347): In der in Schritt a) benutzten Vorentwässerungskolonne (der ersten Destillationskolonne) wird bereits ein Teil des Vinylacetats in ethylacetatfreier Form abgetrennt. Praktisch das gesamte Ethylacetat verbleibt nämlich im Sumpf der Vorentwässerungskolonne, während der in Schritt b) nicht kondensierte Teil des Vinylacetats (der in Schritt e) in Form einer essigsauren Lösung gewonnen wird) und ebenso die etwaige, in Schritt d) abgezogene restliche organische Phase praktisch kein Ethylacetat enthalten und daher eine energetisch aufwendige Befreiung dieser Vinylacetat-Teilströme von Ethylacetat entfällt. Dies wird beim erfindungsgemäßen Verfahren mit einer geringeren Anzahl an Kolonnenböden erreicht als gemäß DE-OS 3 422 575.

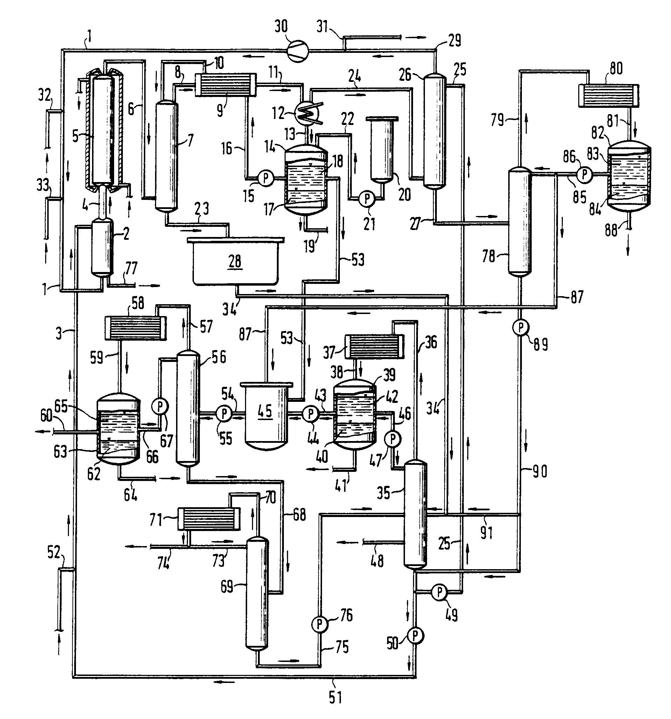

Das erfindungsgemäße Verfahren wird durch Figur 1 erläutert.

Das aus Ethylen, Sauerstoff und CO₂ bestehende Gasgemisch (=Kreisgas) wird über Leitung (1) in einen als Blasensäule ausgebildeten Essigsäureverdampfer (2) geleitet, in dem der Gasstrom mit Essigsäure beladen wird, die über Leitung (3) zugeführt wird. Das den Essigsäureverdampfer (2) verlassende Gasgemisch wird über eine dampfbeheizte Leitung (4) dem Reaktor (5) zugeführt. Dieser besteht aus einem Reaktionsrohr von 5,60 m Länge und 32 mm Innendurchmesser, das von einem Mantel umgeben ist. Die Abführung der Reaktionswärme erfolgt mittels Siedewasser unter Druck in diesem Mantel. Das Reaktionsrohr ist mit dem Katalysator gefüllt. Das den Reaktor (5) verlassende Gasgemisch, das im wesentlichen aus Ethylen, Essigsäure, Vinylacetat, Wasser, Kohlendioxid, Sauerstoff sowie inerten Gasen, wie z.B. Stickstoff und Argon besteht, wird über Leitung (6) in die 1. Destillationskolonne, die Vorentwässerungskolonne (7), eingeleitet. Die Kolonne (7) hat eine Länge von 2,5 m und einen Durchmesser von 50 mm. Sie ist mit Packungen aus gerolltem VA-Drahtnetz (sog. Goodloe-Packungen) gefüllt. Das aus Kolonne (7) über Kopf austretende Gasgemisch gelangt über Leitung (8) in einen Wärmetauscher (9), wo es in Gegenstromwärmeaustausch mit dem Rücklauf gebracht wird, der über Leitung (16) eintritt und über Leitung (10) in Kolonne (7) zurückgeführt wird. Das Gasgemisch gelangt aus dem Wärmetauscher (9) über Leitung (11) in einen wassergekühlten Kondensator (12), in dem es auf etwa 35°C abgekühlt wird. Die dabei verflüssigten Anteile gelangen über Leitung (13) in Behälter (14), wo sie gesammelt werden. Der ein bestimmtes Niveau im Sammelbehälter (14) überschreitende Anteil an Flüssigkeit wird mittels Pumpe (15) über Leitung (16), Wärmetauscher (9) und Leitung (10) in die Vorentwässerungskolonne (7) zurückgepumpt. Nach einiger Zeit trennt sich das im Sammelbehälter (14) anfallende Kondensat in zwei Phasen (17) und (18); von nun an wird die wäßrige Phase (17) über Leitung (19) ausgeschleust und nur die organische Phase (18) wird ganz oder teilweise über Leitung (16), Wärmetauscher (9) und Leitung (10) als Rücklauf auf den Kopf der Vorentwässerungskolonne (7) zurückgepumpt. Aus Vorratsgefäß (20) wird über Pumpe (21) und Leitung (22) Stabilisatorlösung in den Sammelbehälter (14) gepumpt. Die am Sumpf der Vorentwässerungskolonne (7) anfallende Flüssigkeit, die hauptsächlich aus Vinylacetat, Essigsäure und Wasser besteht und fast das gesamte Ethylacetat enthält, wird über Leitung (23) zum Behälter (28) abgezogen. Das den Kondensator (12) über Leitung (24) verlassende Gasgemisch wird in der über Leitung (25) mit Essigsäure beschickten Waschkolonne (26) von nicht kondensierten Vinylacetatanteilen befreit; der Sumpf der Kolonne (26) gelangt über Leitung (27) in die Kolonne (78). Das die Waschkolonne (26) über Leitung (29) verlassende Restgas (Ethylen, nicht umgesetzter Sauerstoff und als Nebenprodukt gebildetes CO₂), wird mittels Kreisgaskompressor (30) über Leitung (1) und den Essigsäureverdampfer (2) zum Reaktor (5) zurückgeführt. Ein Teil des Kreisgases wird zur Ausschleusung von inerten Bestandteilen über Leitung (31) als Abgas entfernt. Über Leitung (32) wird frisches Ethylen und über Leitung (33) frischer Sauerstoff zugeführt.

Das Gemisch aus Behälter (28) wird über Leitung (34) in die zweite Destillationskolonne (35) eingeleitet. Der Kopfdampf der Kolonne (35) wird über Leitung (36) in Kühler (37) geleitet und dort kondensiert. Das über Leitung (38) in Behälter (39) gelangende Kondensat trennt sich in eine wäßrige Phase (40), die über Leitung (41) abgezogen wird und eine organische Phase (42), von der ein Teil über Leitung (43) und Pumpe (44) dem Behälter (45) zugeführt wird, während der andere Teil über Leitung (46) und Pumpe (47) zurückgeführt wird in die Kolonne (35) und dort als Rücklauf dient, um das Durchschlagen von Essigsäure und Ethylacetat zum Kopfprodukt zu verhindern. Das im Gemisch in Behälter (28) enthaltene und über Leitung (34) in Kolonne (35) gelangende Ethylacetat wird aus einer Anreicherungszone oberhalb des Sumpfes der Kolonne (35) über Leitung (48) abgezogen. Das Sumpfprodukt der Kolonne (35) enthält Essigsäure, höchstens 10 Gew.-% Wasser sowie geringe Mengen Hochsieder bzw. Polymere und nur noch Spuren von Vinylacetat und Ethylacetat.

Die wäßrige Essigsäure aus dem Sumpf der Kolonne (35) wird aufgeteilt. Der für die Essigsäurewäsche in Schritt e) benötigte Anteil wird über Pumpe (49) und Leitung (25) der Waschkolonne (26) zugeführt. Der Rest wird über Pumpe (50), Leitung (51) und Leitung (3) wieder dem Essigsäureverdampfer (2) zugeführt. Je nach Auslegung der Waschkolonne (26) und der Temperatur des zu waschenden Gases werden unterschiedliche Mengen an Essigsäure als Waschflüssigkeit benötigt. Die aus dem Sumpf der Kolonne (35) ablaufende wäßrige Essigsäure wird daher entsprechend aufgeteilt. Über die Leitungen (52) und (3) wird frische Essigsäure entsprechend der bei der Reaktion verbrauchten Essigsäuremenge in den Essigsäureverdampfer (2) eingespeist. Das Sumpfprodukt der Kolonne (26) wird über Leitung (27) der dritten Destillationskolonne (78) zugeführt. Ein Teil der organischen Phase (42) aus Behälter (39) wird über Leitung (43) und Pumpe (44) dem Behälter (45) zugeführt. Außerdem gelangt über Leitung (53) der Rest der organischen Phase (18) aus Sammelbehälter (14) in Behälter (45), falls nicht die gesamte organische Phase (18) als Rücklauf in der Vorentwässerungskolonne (7) verwendet wird. Die Flüssigkeit im Behälter (45) wird über Leitung (54) und Pumpe (55) zur vierten Destillationskolonne (56) geführt. Der Kopfdampf der Kolonne (56) wird über Leitung (57) zum Kondensator (58) geführt; das anfallende Kondensat wird über Leitung (59) in den Behälter (63) geleitet. Es trennt sich in Behälter (63) in zwei Phasen, eine wäßrige Phase (62) und eine organische Phase (65). Die wäßrige Phase (62) wird über Leitung (64) abgezogen. Die organische Phase (65) wird über Leitung (66) und Pumpe (67) als Rücklauf zurückgeführt auf den Kopf der Kolonne (56). Ein kleiner Teilstrom der organischen Phase (65) wird über Leitung (60) abgezogen zur Leichtsiederabtrennung. Der praktisch wasserfreie Sumpf der Kolonne (56) wird über Leitung (68) weitergeführt (s.u.). Der Kopfdampf der Kolonne (78) wird über Leitung (79) dem Kondensator (80) zugeführt. Das anfallende Kondensat läuft über Leitung (81) in den Behälter (82). Das Kondensat bildet eine organische Phase (83) und eine wäßrige Phase (84). Die wäßrige Phase (84) wird über Leitung (86) abgezogen. Die organische Phase (83) wird zum Teil über Leitung (85) und Pumpe (86) als Rücklauf zurückgeführt auf den Kopf der Kolonne (78). Ein Teil wird über Leitung (87) abgezogen nach Behälter (45). Das Sumpfprodukt der Kolonne (78) wird im allgemeinen über die Pumpe (89) und Leitung (90) mit dem Sumpfprodukt der Kolonne (35) zusammengeführt, und dann zur Essigsäurewäsche (26) oder zum Essigsäureverdampfer (2) gepumpt. Es kann aber auch über Leitung (91) in Kolonne (35) eingeleitet werden. Das praktisch wasserfreie Vinylacetat, das am Sumpf der Kolonne (56) anfällt, wird über Leitung (68) zur fünften Destillationskolonne (69) geführt. Der Kopfdampf dieser Kolonne geht über Leitung (70) zum Kondensator (71). Das anfallende Kondensat ist praktisch ethylacetatfreies reines Vinylacetat. Über Leitung (73) wird ein sehr kleiner Teil dieses Vinylacetats als Rücklauf in die Kolonne (69) zurückgeleitet. Über Leitung (74) wird reines Vinylacetat abgezogen. Das Sumpfprodukt der Kolonne (69), das Polymere, Hochsieder und Vinylacetat/Ethylacetat/Essigsäure enthält, wird über Leitung (75) und Pumpe (76) zurückgeführt zur Kolonne (35). Aus dem Essigsäureverdampfer (2), in den letztlich alle Hochsieder und Polymeren zurückgeführt werden, wird über Leitung (77) ein Teilstrom zur Polymerenausschleusung abgezogen.

### Beispiel

Der folgende Versuch wurde in der oben beschriebenen und in Figur 1 dargestellten Apparatur durchgeführt. Der Reaktor (5) war gefüllt mit 4,4 l eines bekannten Vinylacetat-Katalysators, der 2,3 Gew.-% Palladium, 2 Gew.-% Kalium und 1,9 Gew.-% Cadmium, jeweils in Form ihrer Acetate auf einem Kieselsäureträger (Kugeln von 4 - 6 mm Durchmesser) enthielt. In den Essigsäureverdampfer (2) wurden pro Stunde 12 Nm³ eines Gemisches eingeleitet, das ca. 69 Vol.-% Ethylen, 24 Vol.-% Kohlendioxid und 7 Vol.-% Sauerstoff enthielt. Dem Essigsäureverdampfer (2) wurde über Leitung (3) soviel Essigsäure zugeführt, daß in ihm pro Stunde 4,83 kg Essigsäure verdampften. Zur Hochsieder- und Polymerenausschleusung wurden pro Stunde 0,5 kg Material aus dem Essigsäureverdampfer (2) über Leitung (77) abgezogen. Das in den Reaktor einströmende Gas wurde in Leitung (4) auf 155°C vorgeheizt. Am Eingang des Reaktors (5) wurde ein Überdruck von 8 bar (9 bar absolut) eingestellt, die Temperatur am Ausgang des Reaktors wurde über den Druck auf der Siedewasserkühlung im Reaktoraußenmantel auf 160°C eingestellt. Die Temperatur der Reaktionsgase am Eingang der Vorentwässerungskolonne (7) betrug wegen der Wärmestrahlung der Leitung (6) nur noch 130°C. Das die Vorentwässerungskolonne (7) über Kopf verlassende Gasgemisch wurde im Kondensator (12) auf 35°C abgekühlt. Im Behälter (14) fielen pro Stunde 9 kg organische Phase (18) an, die über Pumpe (15) und Wärmetauscher (9) in die Vorentwässerungskolonne (7) zurückgeführt wurden. Aus dem Behälter (14) wurden pro Stunde 350 g einer wäßrigen Phase (17) abgezogen, die 3 Gew.-% Vinylacetat, 0,1 Gew.-% Essigsäure und 0,05 Gew.-% Acetaldehyd enthielt. Am Sumpf der Vorentwässerungskolonne (7), in der sich eine Kopftemperatur von 80°C und eine Sumpftemperatur von 90°C einstellt, fielen pro Stunde 4 kg eines Gemisches aus 74 Gew.-% Essigsäure, 7,5 Gew.-% Wasser, 17,5 Gew.-% Vinylacetat, 0,05 Gew.-% Ethylidendiacetat, 0,06 Gew.-% Ethylacetat, 0,1 Gew.-% Acetaldehyd und 0,05 Gew.-% Hochsieder sowie Polymere an. Zur Stabilisierung wurden pro Stunde 15 ml einer Lösung von 2,5 Gew.-% p-Benzochinon in Vinylacetat aus Vorratsgefäß (20) zum Behälter (14) gepumpt.

Das Restgas aus dem Kondensator (12) wurde über Leitung (24) in die Waschkolonne (26) eingeleitet. Auf den Kopf der Waschkolonne (26) wurden pro Stunde 3,1 kg wasserhaltiger Essigsäure aus dem Sumpf der Kolonne (35) über Leitung (25) gepumpt. Am Sumpf der Waschkolonne (26) fielen pro Stunde 5 kg eines Gemisches aus 57,6 Gew.-% Essigsäure, 5,1 Gew.-% Wasser, 37 Gew.-% Vinylacetat, 0,02 Gew.-% Acetaldehyd und 30 Gew.-ppm Ethylacetat an.

Das die Waschkolonne (26) verlassende Gas wurde über Leitung (29) und den Kreisgaskompressor (30) zum Essigsäureverdampfer (2) zurückgeführt. Bei der Reaktion verbrauchtes Ethylen und verbrauchter Sauerstoff wurden durch Zufuhr von Frischethylen - Leitung (32) - und Frischsauerstoff - Leitung (33) - im Kreisgas ersetzt. Das bei der Reaktion als Nebenprodukt gebildete CO₂ wurde als Abgas über Leitung (31) aus dem Kreisgas entfernt; die Abgasmenge wurde so eingestellt, daß im Kreisgas eine CO₂-Konzentration von 24 Vol.-% aufrechterhalten wurde.

Das Sumpfprodukt der Vorentwässerungskolonne (7) wurde über Leitung (23) zum Vorratsbehälter (28) abgezogen und von dort über Leitung (34) in die zweite Destillationskolonne (35) eingeleitet. Kolonne (35) war als vakuumummantelte Glaskolonne mit einem Innendurchmesser von 50 mm, einer Länge von 6 m und 80 Glockenböden ausgelegt und hatte eine elektrische Sumpfbeheizung.

Pro Stunde wurden auf den 40. Boden der Kolonne (35) 4 kg der Mischung aus Behälter (28) eingeleitet. Die Sumpfbeheizung wurde so eingestellt, daß die Gesamtdestillatmenge 5,3 kg/h betrug. Es fielen im Phasentrenner (39) 177 g/h wäßrige Phase (40) an, die abgezogen wurde. Von der organischen Phase (42) wurde ein solcher Teil über Leitung (43) zum Behälter (45) gepumpt (etwa 0,7 kg/h), daß die Temperatur in Kolonne (35) am 25. Boden 112°C betrug. Die größere Menge der organischen Phase (42) wurde als Rücklauf über Leitung (46) zurückgeführt in die Kolonne (35). Die nach Behälter (45) abgezogene organische Phase (42) enthielt neben Vinylacetat noch 0,05 Gew.-% Essigsäure, 0,2 Gew.-% Acetaldehyd, 0,015 Gew.-% Ethylacetat und 1,3 Gew.-% Wasser.

Am 20. Boden der Kolonne (35) befand sich ein Seitenabzug (48), aus dem pro Stunde 50 g Material abgezogen wurde, das etwa 5 Gew.-% Ethylacetat, 2 Gew.-% Vinylacetat. 12 Gew.-% Wasser und 81 Gew.-% Essigsäure enthielt. Die am Sumpf von Kolonne (35) anfallende Essigsäure enthielt etwa 4 Gew.-% Wasser; 3,1 kg davon wurden über Leitung (25) in die Waschkolonne (26) geleitet, der Rest wurde über Pumpe (50) und die Leitungen (51) und (3) zum Essigsäureverdampfer (2) geführt.

Im Behälter (45) fielen pro Stunde 2,7 kg Material an, das neben Vinylacetat 1,3 Gew.-% Wasser, 0,04 Gew.-% Acetaldehyd und 0,01 Gew.-% Ethylacetat enthielt.

Dem Behälter (45) wurden folgende Einzelströme zugeführt: 0,7 kg aus dem Destillat von Kolonne (35), 1,85 kg aus dem Destillat von Kolonne (78) und 0,18 kg aus dem Destillat der Vorentwässerungskolonne (7).

Dieses Material wurde in der vierten Destillationskolonne (56) weiter destilliert. Die Kolonne bestand aus Glas, besaß einen Vakuummantel und eine elektrische Sumpfbeheizung. Sie hatte 40 Böden, der Zulauf befand sich auf Boden 30. Die Sumpfbeheizung wurde so eingestellt, daß im Phasentrenner (62) pro Stunde 2 kg Destillat anfielen. Die Menge der unteren wäßrigen Phase (63) betrug 35 g, sie wurde über Leitung (64) abgezogen. Von der organischen Phase (65), die 5 Gew.-% Acetaldehyd enthielt, wurden über Leitung (60) 20 g abgezogen. Die Hauptmenge der organischen Phase wurde über Leitung (66) und Pumpe (67) zurückgepumpt auf den Kopf von Kolonne (56). Der Sumpfablauf von Kolonne (56) wurde in der fünften Destillationskolonne (69) weiter aufgearbeitet. Die Kolonne (69) bestand aus Glas, besaß einen Vakuummantel und eine elektrische Sumpfbeheizung. Sie hatte einen Innendurchmesser von 50 mm und 15 Böden. Bei einem Rücklaufverhältnis von 0,2 erhielt man über Leitung (74) 2,4 kg/h reines Vinylacetat, das weniger als 50 Gew.-ppm Essigsäure, ca. 100 Gew.-ppm Wasser und 40 Gew.-ppm Ethylacetat enthielt. Aus dem Sumpf von Kolonne (69) wurden mit Pumpe (76) über Leitung (75) 250 g zurückgepumpt zur Kolonne (35). Aus dem Sumpf der Essigsäurewäsche wurden über Leitung (27) pro Stunde 5 kg eines Gemisches, bestehend aus 57,6 Gew.-% Essigsäure, 37 Gew.-% Vinylacetat, 5,1 Gew.-% Wasser, 0,02 Gew.-% Acetaldehyd und 0,003 Gew.-% Ethylacetat zu der dritten Destillationskolonne (78) geleitet.

Die Kolonne (78) war als vakuumummantelte Glaskolonne ausgelegt und hatte eine elektrische Sumpfbeheizung. Ihr Innendurchmesser betrug 50 mm; sie hatte 40 Böden. Das Destillat wurde über Leitung (79) zum Kondensator (80) geführt. Das Kondensat gelangte über Leitung (81) in die Vorlage (82). Das Kondensat in Vorlage (82) trennte sich in zwei Phasen. Pro Stunde fielen 5 kg organische Phase (83) und 166 g wäßrige Phase (84) an. Die wäßrige Phase wurde über Leitung (88) abgezogen. Von der organischen Phase (83) wurden über Leitung (85) und Pumpe (86) 3,33 kg/h als Rücklauf auf den Kopf von Kolonne (78) geführt, 1,85 kg/h wurden über Leitung (87) zum Behälter (45) gepumpt. Über Pumpe (89) und Leitung (90) wurde das Sumpfprodukt von Kolonne (78) zum Sumpfablauf von Kolonne (35) gepumpt.

## Patentansprüche

1. Verfahren zur Isolierung von Vinylacetat aus einem Vinylacetat, Ethylacetat, Essigsäure, Wasser und Kohlendioxid enthaltenden Gasgemisch, welches bei der Reaktion von Ethylen mit Essigsäure und Sauerstoff in einer Reaktionszone in der Gasphase an Palladium oder Palladiumverbindungen enthaltenden Katalysatoren gebildet wird, wobei man
a) das aus der Reaktionszone austretende Gasgemisch in eine erste Destillationskolonne einleitet,
b) das am Kopf der ersten Destillationskolonne austretende Gasgemisch auf -20 bis +50°C abkühlt, wobei sich das anfallende Kondensat in eine wäßrige und eine organische Phase trennt,
c) die in Schritt b) gebildete wäßrige Phase abzieht,
d) die in Schritt b) gebildete organische Phase ganz oder teilweise als Rücklauf auf den Kopf der in Schritt a) benutzten ersten Destillationskolonne zurückführt und einen etwaigen nicht als Rücklauf verwendeten Teil der organischen Phase abzieht,
e) das im Schritt b) nicht kondensierte, Vinylacetat enthaltende Gas in einer Waschkolonne mit mindestens 90 %iger wäßriger Essigsäure wäscht und dabei am Sumpf eine vinylacetathaltige essigsaure Lösung gewinnt,
f) das Vinylacetat, Ethylacetat, Essigsäure und Wasser enthaltende Sumpfprodukt von Schritt a) einer zweiten Destillationskolonne zuführt und aus einer Anreicherungszone oberhalb von deren Sumpf einen ethylacetathaltigen Seitenstrom abzieht,
g) das Essigsäure und Wasser enthaltende Sumpfprodukt von Schritt f) ganz oder teilweise zur Gaswäsche in Schritt e) benutzt,
h) den Kopfdampf von Schritt f) abkühlt, wobei sich das anfallende Kondensat in eine wäßrige und eine organische Phase trennt,
i) die in Schritt h) gebildete wäßrige Phase abzieht,
k) einen Teil der in Schritt h) gebildeten organischen Phase als Rücklauf auf den Kopf der in Schritt f) benutzten zweiten Destillationskolonne zurückführt,
l) den restlichen Teil der in Schritt h) gebildeten organischen Phase abzieht,
dadurch gekennzeichnet, daß man
m) das Sumpfprodukt der in Schritt e) benutzten Waschkolonne in eine dritte Destillationskolonne einleitet,
n) das Sumpfprodukt der in Schritt m) benutzten dritten Destillationskolonne in die in Schritt f) benutzte zweite Destillationskolonne oder in die in Schritt e) benutzte Waschkolonne oder in die Reaktionszone zurückführt,
o) den Kopfdampf von Schritt m) abkühlt, wobei sich das anfallende Kondensat in eine wäßrige und eine organische Phase trennt,
p) die in Schritt o) gebildete wäßrige Phase abzieht,
q) einen Teil der in Schritt o) gebildeten organischen Phase als Rücklauf auf den Kopf der in Schritt m) benutzten dritten Destillationskolonne zurückführt,
r) den restlichen Teil der in Schritt o) gebildeten organischen Phase zusammen mit der in Schritt l) abgezogenen restlichen organischen Phase und zusammen mit der etwaigen, in Schritt d) abgezogenen restlichen organischen Phase in eine vierte Destillationskolonne einleitet,
s) den Kopfdampf von Schritt r) abkühlt, wobei sich das anfallende Kondensat in eine wäßrige und eine organiche Phase trennt,
t) die in Schritt s) gebildete wäßrige Phase abzieht,
u) die in Schritt s) gebildete organische Phase ganz oder teilweise als Rücklauf auf den Kopf der in Schritt r) benutzten vierten Destillationskolonne zurückführt und einen etwaigen nicht als Rücklauf verwendeten Teil der organischen Phase zur Leichtsiederabtrennung abzieht,
v) das Sumpfprodukt von Schritt r) in eine fünfte Destillationskolonne einleitet,
w) am Kopf der in Schritt v) verwendeten fünften Destillationskolonne reines Vinylacetat abzieht.

2. Verfahren nach Anspruch 1, dadurch gekennzeichnet, daß man in Schritt a) das aus der Reaktionszone austretende Gasgemisch zunächst im Gegenstromwärmeaustausch mit dem kälteren Kreisgas auf etwa 115 - 130°C abkühlt und erst dann in die erste Destillationskolonne einleitet.

3. Verfahren nach Anspruch 1 oder 2, dadurch gekennzeichnet, daß man die Abkühlungstemperatur in Schritt b) und den als Rücklauf in Schritt d) benutzten Anteil der in b) gebildeten organischen Phase derart wählt, daß möglichst wenig Vinylacetat, aber möglichst das gesamte Ethylacetat im Sumpfprodukt von Schritt a) enthalten sind.

4. Verfahren nach einem der Ansprüche 1 bis 3, dadurch gekennzeichnet, daß man in Schritt k) nur soviel von der in Schritt h) gebildeten organischen Phase als Rücklauf zurückführt, daß der Kopfdampf der zweiten Destillationskolonne möglichst wenig Essigsäure und Ethylacetat enthält und denjenigen Teil der organischen Phase, der nicht für diesen Zweck benötigt wird, gemäß Schritt r) in die vierte Destillationskolonne einleitet.

5. Verfahren nach einem der Ansprüche 1 bis 4, dadurch gekennzeichnet, daß man in Schritt q) soviel von der in Schritt o) gebildeten organischen Phase als Rücklauf auf den Kopf der dritten Destillationskolonne zurückführt, daß in deren Sumpf möglichst wenig Vinylacetat erhalten ist.

6. Verfahren nach einem der Ansprüche 1 bis 5, dadurch gekennzeichnet, daß man in Schritt u) die in Schritt s) gebildete organische Phase nicht ganz als Rücklauf für die vierte Destillationskolonne benutzt, sondern einen zur Leichtsiederabtrennung ausreichenden Teil abzieht.

## Claims

1. A process for the isolation of vinyl acetate from a gas mixture containing vinyl acetate, ethyl acetate, acetic acid, water and carbon dioxide which is formed in the reaction of ethylene with acetic acid and oxygen in a reaction zone in the gas phase over catalysts containing palladium or palladium compounds, in which process
a) the gas mixture leaving the reaction zone is passed into a first distillation column,
b) the gas mixture leaving at the head of the first distillation column is cooled to - 20 to + 50°C, in the course of which the condensate formed separates into an aqueous and an organic phase,
c) the aqueous phase formed in step b) is removed,
d) the organic phase formed in step b) is reintroduced completely or in part as reflux at the head of the first distillation column used in step a) and any portion of the organic phase not used as reflux is removed,
e) the gas not condensed in step b) and containing vinyl acetate is washed in a washing column with at leat 90 % aqueous acetic acid to give an acetic acid solution containing vinyl acetate at the bottom,
f) the bottom product of step a) containing vinyl acetate, ethyl acetate, acetic acid and water is passed to a second distillation column and a sidestream containing ethyl acetate is removed from a concentration zone above the bottom thereof,
g) the bottom product of step f) containing acetic acid and water is used completely or in part for the gas washing in step e),
h) the head vapor of step f) is cooled, in the course of which the condensate formed separates into an aqueous and an organic phase,
i) the aqueous phase formed in step h) is removed,
k) a portion of the organic phase formed in step h) is reintroduced as reflux at the head of the second distillation column used in step f),
l) the remaining portion of the organic phase formed in step h) is removed,
which comprises
m) introducing the bottom product of the washing column used in step e) into a third distillation column,
n) reintroducing the bottom product of the third distillation column used in step m) into the second distillation column used in step f) or into the washing column used in step e) or into the reaction zone,
o) cooling the head vapor of step m) in the course of which the condensate formed separates into an aqueous and an organic phase,
p) removing the aqueous phase formed in step o),
q) re-introducing a portion of the organic phase formed in step o) as reflux at the head of the third distillation column used in step m),
r) introducing the remaining portion of the organic phase formed in step o) together with the remaining organic phase removed in step 1) and together with any remaining organic phase removed in step d) into a fourth distillation column,
s) cooling the head vapor of step r), in the course of which the condensate formed separates into an aqueous and an organic phase,
t) removing the aqueous phase formed in step s),
u) re-introducing the organic phase formed in step s) completely or in part as reflux at the head of the fourth distillation column used in step r) and removing any portion of the organic phase not used as reflux, in order to separate off the low-boiling components,
v) introducing the bottom product from step r) into a fifth distillation column,
w) removing pure vinyl acetate at the head of the fifth distillation column used in step v).

2. The process as claimed in claim 1, wherein in step a) the gas mixture leaving the reaction zone is first cooled to about 115-130°C by counter-current heat exchange with the colder recycled gas and only then introduced into the first distillation column.

3. The process as claimed in claim 1 or 2, wherein the cooling temperature in step b) and the portion of the organic phase formed in b) which is used as reflux in step d) are selected such that a minimum amount of vinyl acetate but, if possible, all of the entire ethyl acetate is present in the bottom product of step a).

4. The process as claimed in any of claims 1 to 3, wherein in step k) only such an amount of the organic phase formed in step h) is re-introduced as reflux that the head vapor of the second distillation column contains a minimum amount of acetic acid and ethyl acetate and the portion of the organic phase which is not required for this purpose is introduced into the fourth distillation column according to step r).

5. The process as claimed in any of claims 1 to 4, wherein in step q) such an amount of the organic phase formed in step o) is reintroduced as reflux at the head of the third distillation column that a minimum amount of vinyl acetate is obtained in the bottom thereof.

6. The process as claimed in any of claims 1 to 5, wherein in step u) the organic phase formed in step s) is not used in its entirety as reflux for the fourth distillation column but a portion sufficient for separating off the low-boiling components is removed.

## Revendications

1. Procédé d'isolement d'acétate de vinyle à partir d'un mélange gazeux contenant de l'acétate de vinyle, de l'acétate d'éthyle, de l'acide acétique, de l'eau et du dioxyde de carbone, formé par la réaction d'éthylène avec de l'acide acétique et de l'oxygène dans une zone de réaction en phase gazeuse sur des catalyseurs contenant du palladium ou des composés du palladium, dans lequel
a) on introduit le mélange gazeux issu de la zone de réaction dans une première colonne de distillation,
b) on refroidit à une température de -20 à +50°C le mélange gazeux sortant en tête de la première colonne de distillation, ce qui fait que le condensat formé se sépare en une phase aqueuse et une phase organique,
c) on soutire la phase aqueuse formée dans l'étape b),
d) on renvoie tout ou partie de la phase organique formée dans l'étape b) sous forme de reflux en tête de la première colonne de distillation utilisée dans l'étape a), et on soutire l'éventuelle partie de la phase organique non utilisée comme reflux,
e) on lave dans une colonne de lavage le gaz non condensé dans l'étape b), contenant de l'acétate de vinyle, avec de l'acide acétique aqueux à au moins 90 %, et on obtient ainsi en bas de colonne une solution d'acide acétique contenant de l'acétate de vinyle,
f) on envoie le produit de bas de colonne de l'étape a), contenant de l'acétate de vinyle, de l'acétate d'éthyle, de l'acide acétique et de l'eau dans une deuxième colonne de distillation et on soutire à partir d'une zone de concentration située au-dessus du bas de colonne un courant latéral contenant de l'acétate d'éthyle,
g) on utilise en totalité ou en partie le produit de bas de colonne de l'étape f), contenant de l'acide acétique et de l'eau, pour le lavage du gaz dans l'étape e),
h) on refroidit la vapeur de tête de l'étape f), ce qui fait que le condensat formé se sépare en une phase aqueuse et une phase organique,
i) on soutire la phase aqueuse formée dans l'étape h),
k) on renvoie une partie de la phase organique formée dans l'étape h) sous forme de reflux en tête de la deuxième colonne de distillation utilisée dans l'étape f),
l) on soutire la partie restante de la phase organique formée dans l'étape h),
caractérisé en ce que
m) on fait passer le produit de bas de colonne de la colonne de lavage utilisée dans l'étape e) dans une troisième colonne de distillation,
n) on renvoie le produit de bas de colonne de la troisième colonne de distillation utilisée dans l'étape m) dans la deuxième colonne de distillation utilisée dans l'étape f) ou dans la colonne de lavage utilisée dans l'étape e) ou dans la zone de réaction,
o) on refroidit la vapeur de tête de l'étape m), ce qui fait que le condensat formé se sépare en une phase aqueuse et une phase organique,
p) on soutire la phase aqueuse formée dans l'étape o),
q) on renvoie sous forme de reflux une partie de la phase organique formée dans l'étape o) en tête de la troisième colonne de distillation utilisée dans l'étape m),
r) on fait passer la partie restante de la phase organique formée dans l'étape o), avec la phase organique restante soutirée dans l'étape l) et avec l'éventuelle phase organique restante soutirée dans l'étape d), dans une quatrième colonne de distillation,
s) on refroidit la vapeur de tête de l'étape r), ce qui fait que le condensat formé se sépare en une phase aqueuse et une phase organique,
t) on soutire la phase aqueuse formée dans l'étape s),
u) on renvoie tout ou partie de la phase organique formée dans l'étape s) sous forme de reflux en tête de la quatrième colonne de distillation utilisée dans l'étape r), et on soutire l'éventuelle partie de la phase organique non utilisée comme reflux pour séparer les produits de bas point d'ébullition,
v) on fait passer le produit de bas de colonne de l'étape r) dans une cinquième colonne de distillation,
w) on soutire de l'acétate de vinyle pur en tête de la cinquième colonne de distillation utilisée dans l'étape v).

2. Procédé selon la revendication 1, caractérisé en ce que, dans l'étape a), on refroidit d'abord à une température d'environ 115 - 130°C le mélange gazeux sortant de la zone de réaction par un échange de chaleur à contre-courant avec le gaz de circulation plus froid, et on ne le fait passer qu'ensuite dans la première colonne de distillation.

3. Procédé selon la revendication 1 ou 2, caractérisé en ce que l'on choisit la température de refroidissement dans l'étape b) et la fraction de la phase organique formée dans l'étape b) utilisée comme reflux dans l'étape d) de façon que le produit de bas de colonne de l'étape a) contienne aussi peu que possible d'acétate de vinyle, mais autant que possible tout l'acétate de vinyle.

4. Procédé selon l'une des revendications 1 à 3, caractérisé en ce que, dans l'étape k), on ne renvoie comme reflux que la quantité de phase organique formée dans l'étape h) nécessaire pour que la vapeur de tête de la deuxième colonne de distillation contienne aussi peu que possible d'acide acétique et d'acétate d'éthyle, et on fait passer la fraction de la phase organique qui n'est pas nécessaire à cet effet dans la quatrième colonne de distillation selon l'étape r).

5. Procédé selon l'une des revendications 1 à 4, caractérisé en ce que, dans l'étape q), on renvoie comme reflux en tête de la troisième colonne de distillation la quantité de phase organique formée dans l'étape o) nécessaire pour que son produit de bas de colonne contienne aussi peu que possible d'acétate de vinyle.

6. Procédé selon l'une des revendications 1 à 5, caractérisé en ce que, dans l'étape u), on n'utilise pas toute la phase organique formée dans l'étape s) comme reflux pour la quatrième colonne de distillation, mais on en soutire une fraction suffisante pour séparer les produits de bas point d'ébullition.
